# EUROPEAN PATENT SPECIFICATION

(11) **EP 0 431 880 B1**
(45) Date of publication and mention of the grant of the patent: **21.02.1996**
(21) Application number: 90313129.0
(22) Date of filing: 04.12.1990
(51) Int. Cl.: G01N 33/53, G01N 33/94

(54) **Mutant fungal strain detection**
Mutantschimmelstammnachweis
Détection de lignée de champignon mutage

(30) Priority: 05.12.1989 GB 8927480
(43) Date of publication of application: 12.06.1991
(73) Proprietor: Delta Biotechnology Limited, Nottingham NG7 1FD (GB)
(72) Inventor: Goodey, Andrew Robert, Mapperley Park, Nottingham NG3 5DS (GB); Belfield, Graham Paul, Beeston, Nottingham NG9 2GT (GB); Sleep, Darrell, West Bridgeford, Nottingham NG2 5DS (GB)
(74) Representative: Bassett, Richard Simon

(56) References cited:
- EP-A- 0 201 208
- EP-A- 0 322 094
- US-A- 3 709 661
- US-A- 4 430 428
- US-A- 4 882 273
- YEAST, vol. 6, "Special Issue", 1990, John Wiley & Sons Ltd, New York, NY (US); D. SLEEP et al., p. S-420/

### INFORMATION FOR SEQ ID NO:1:

(i) SEQUENCE CHARACTERISTICS:
   (A) LENGTH: 1440 base pairs
   (B) TYPE: nucleic acid
   (C) STRANDEDNESS: double
   (D) TOPOLOGY: linear
(ii) MOLECULE TYPE: DNA (genomic)
(iii) HYPOTHETICAL: N
(iv) ANTI-SENSE: N
(vi) ORIGINAL SOURCE:
   (A) ORGANISM: Saccharomyces cerevisiae
(xi) SEQUENCE DESCRIPTION: SEQ ID NO:1:
   AAGCTTACTG CTGACCTTTG CCTTGGTCCT TGTACTTGTC TCGATTGCTG NNACCATTAA     60
   CGGTTTTTTG TTCTTACTTA ATCTTTTTTT TCCCGACGAC GCAAAATGTC AGAAAAGATA    120
   TAAANAGAGC AACGTTTCCT GCTACATGAA ACACGCTGGC ATATAGAAGA ACTTCAACGA    180
   GCGAGGCTCT CTGAACAATC TGCTAATCGT GCAAGTAAGG GTTCTCGTGA CAGTTCACCG    240
   TGTATAAACA AGNNNAAACA GATAAACAGT CAGATATGCT TAATTTTTAA CTGTAGGCTG    300
   TGCCGACATG CCATTTTATG CGGCTCACTT CTAACAAAAG TGACCATGAC GCACAAGCAA    360
   GCAAACAGCC AAGTAAGGAA GCAGACAGCT TCACAGCACA CACACACCGT TGTGTACGAA    420
   AATCCTCTGC AGAAGAGTGT ATATGCCACT CATCCACACT TCCGCAGCGC AGTACGTAAT    480
   GCGGTATCGT GAAAGCGAAA AAAAAACTAA CAGTAGATAA GACAGATAGA CAGATAGAGA    540
   TGGACGAGAA ACAGGGGGGG AGAAAAGGGG AAAAGAGAAG GAAAGAAAGA CTCATCTATC    600
   GCAGATAAGA CAATCAACCC TCATGGCGCC TCCAACCACC ATCCGCACTA GGGACCAAGC    660
   GCTCGCACCG TTAGCAACGC TTGACTCACA AACCAACTGC CGGCTGAAAG AGCTTGTGCA    720
   ATGGGAGTGC CAATTCAAAG GAGCCGAATA CGTCTGCTCG CCTTTTAAGA GGCTTTTTGA    780
   ACACTGCATT GCACCCGACA AATCAGCCAC TAACTACGAG GTCACGGACA CATATACCAA    840
   TAGTTAAAAA TTACATATAC TCTATATAGC ACAGTAGTGT GATAAATAAA AAATTTTGCC    900
   AAGACTTTTT TAAACTGCAC CCGACAGATC AGGTCTGTGC CTACTATGCA CTTATGCCCG    960
   GGGTCCCGGG AGGAGAAAAA ACGAGGGCTG GGAAATGTCC GTGGACTTTA AACGCTCCGG   1020
   GTTAGCAGAG TAGCAGGGCT TTCGGCTTTG GAAATTTAGG TGACTTGTTG AAAAAGCAAA   1080
   ATTTGGGCTC AGTAATGCCA CTGCAGTGGC TTATCACGCC AGGACTGCGG GAGTGGCGGG   1140
   GGCAAACACA CCCGCGATAA AGAGCGCGAT GAATATAAAA GGGGGCCAAT GTTACGTCCC   1200
   GTTATATTGG AGTTCTTCCC ATACAAACTT AAGAGTCCAA TTAGCTTCAT CGCCAATAAA   1260
   AAAACAAACT AAACCTAATT CTAACAAGCA AAGATGAAGT TAGAAAATAC TCTATTTACA   1320
   CTCGGTGCCC TAGGGAGCAT CTCTGCTGCT TTGGTCATCC CAAATCTTGA AAATGCCGCC   1380
   GACCACCACG AACTGATTAA CAAGGAAGAT CACCACGAGA GACCCAGAAA AGTGGAATTC   1440

### INFORMATION FOR SEQ ID NO:8:

(i) SEQUENCE CHARACTERISTICS:
   (A) LENGTH: 1412 base pairs
   (B) TYPE: nucleic acid
   (C) STRANDEDNESS: double
   (D) TOPOLOGY: linear
(ii) MOLECULE TYPE: cDNA to mRNA
(iii) HYPOTHETICAL: N
(iv) ANTI-SENSE: N
(vi) ORIGINAL SOURCE:
   (A) ORGANISM: Homo sapiens
(ix) FEATURE:
   (A) NAME/KEY: exon
   (B) LOCATION: 1..1412
   (D) OTHER INFORMATION:
(xi) SEQUENCE DESCRIPTION: SEQ ID NO:8:
   AGCAACTCAG AGAATAACCA GAGAACAACC AGATTGAAAC AATGGAGGAT CTTTGTGTGG      60
   CAAACACACT CTTTGCCCTC AATTTATTCA AGCATCTGGC AAAAGCAAGC CCCACCCAGA     120
   ACCTCTTCCT CTCCCCATGG AGCATCTCGT CCACCATGGC CATGGTCTAC ATGGGCTCCA     180
   GGGGCAGCAC CGAAGACCAG ATGGCCAAGG TGCTTCAGTT TAATGAAGTG GGAGCCAATG     240
   CAGTTACCCC CATGACTCCA GAGAACTTTA CCAGCTGTGG GTTCATGCAG CAGATCCAGA     300
   AGGGTAGTTA TCCTGATGCG ATTTTGCAGG CACAAGCTGC AGATAAAATC CATTCATCCT     360
   TCCGCTCTCT CAGCTCTGCA ATCAATGCAT CCACAGGGAA TTATTTACTG GAAAGTGTCA     420
   ATAAGCTGTT TGGTGAGAAG TCTGCGAGCT TCCGGGAAGA ATATATTCGA CTCTGTCAGA     480
   AATATTACTC CTCAGAACCC CAGGCAGTAG ACTTCCTAGA ATGTGCAGAA GAAGCTAGAA    540
   AAAAGATTAA TTCCTGGGTC AAGACTCAAA CCAAAGGCAA AATCCCAAAC TTGTTACCTG    600
   AAGGTTCTGT AGATGGGGAT ACCAGGATGG TCCTGGTGAA TGCTGTCTAC TTCAAAGGAA    660
   AGTGGAAAAC TCCATTTGAG AAGAAACTAA ATGGGCTTTA TCCTTTCCGT GTAAACTCGG    720
   CTCAGCGCAC ACCTGTACAG ATGATGTACT TGCGTGAAAA GCTAAACATT GGATACATAG    780
   AAGACCTAAA GGCTCAGATT CTAGAACTCC CATATGCTGG AGATGTTAGC ATGTTCTTGT    840
   TGCTTCCAGA TGAAATTGCC GATGTGTCCA CTGGCTTGGA GCTGCTGGAA AGTGAAATAA    900
   CCTATGACAA ACTCAACAAG TGGACCAGCA AAGACAAAAT GGCTGAAGAT GAAGTTGAGG    960
   TATACATACC CCAGTTCAAA TTAGAAGAGC ATTATGAACT CAGATCCATT CTGAGAAGCA   1020
   TGGGCATGGA GGACGCCTTC AACAAGGGAC GGGCCAATTT CTCAGGGATG TCGGAGAGGA   1080
   ATGACCTGTT TCTTTCTGAA GTGTTCCACC AAGCCATGGT GGATGTGAAT GAGGAGGGCA   1140
   CTGAAGCAGC CGCTGGCACA GGAGGTGTTA TGACAGGGAG AACTGGACAT GGAGGCCCAC   1200
   AGTTTGTGGC AGATCATCCT TTTCTTTTTC TTATTATGCA TAAGATAACC AACTGCATTT   1260
   TATTTTTCGG CAGATTTTCC TCACCCTAAA ACTAAGCGTG CTGCTTCTGC AAAAGATTTT   1320
   TGTAGATGAG CTGTGTGCCT CAGAATTGCT ATTTCAAATT GCCAAAAATT TAGAGATGTT   1380
   TTCTACATAT TTCTGCTCTT CTGAACAACT TC                                 1412

### INFORMATION FOR SEQ ID NO:19:

(i) SEQUENCE CHARACTERISTICS:
   (A) LENGTH: 1352 base pairs
   (B) TYPE: nucleic acid
   (C) STRANDEDNESS: double
   (D) TOPOLOGY: linear
(ii) MOLECULE TYPE: cDNA to mRNA
(iii) HYPOTHETICAL: N
(iv) ANTI-SENSE: N
(vi) ORIGINAL SOURCE:
   (A) ORGANISM: Homo sapiens
(xi) SEQUENCE DESCRIPTION: SEQ ID NO:19:
   CTGGGACAGT GAATCGACAA TGCCGTCTTC TGTCTCGTGG GGCATCCTCC TGCTGGCAGG     60
   CCTGTGCTGC CTGGTCCCTG TCTCCCTGGC TGAGGATCCC CAGGGAGATG CTGCCCAGAA    120
   GACAGATACA TCCCACCATG ATCAGGATCA CCCAACCTTC AACAAGATCA CCCCCAACCT    180
   GGCTGAGTTC GCCTTCAGCC TATACCGCCA GCTGGCACAC CAGTCCAACA GCACCAATAT    240
   CTTCTTCTCC CCAGTGAGCA TCGCTACAGC CTTTGCAATG CTCTCCCTGG GGACCAAGGC    300
   TGACACTCAC GATGAAATCC TGGAGGGCCT GAATTTCAAC CTCACGGAGA TTCCGGAGGC    360
   TCAGATCCAT GAAGGCTTCC AGGAACTCCT CCGTACCCTC AACCAGCCAG ACAGCCAGCT    420
   CCAGCTGACC ACCGGCAATG GCCTGTTCCT CAGCGAGGGC CTGAAGCTAG TGGATAAGTT    480
   TTTGGAGGAT GTTAAAAAGT TGTACCACTC AGAAGCCTTC ACTGTCAACT TCGGGGACAC    540
   CGAAGAGGCC AAGAAACAGA TCAACGATTA CGTGGAGAAG GGTACTCAAG GGAAAATTGT    600
   GGATTTGGTC AAGGAGCTTG ACAGAGACAC AGTTTTTGCT CTGGTGAATT ACATCTTCTT    660
   TAAAGGCAAA TGGGAGAGAC CCTTTGAAGT CAAGGACACC GAGGAAGAGG ACTTCCACGT    720
   GGACCAGGTG ACCACCGTGA AGGTGCCTAT GATGAAGCGT TTAGGCATGT TTAACATCCA    780
   GCACTGTAAG AAGCTGTCCA GCTGGGTGCT GCTGATGAAA TACCTGGGCA ATGCCACCGC    840
   CATCTTCTTC CTGCCTGATG AGGGGAAACT ACAGCACCTG GAAAATGAAC TCACCCACGA    900
   TATCATCACC AAGTTCCTGG AAAATGAAGA CAGAAGGTCT GCCAGCTTAC ATTTACCCAA    960
   ACTGTCCATT ACTGGAACCT ATGATCTGAA GAGCGTCCTG GGTCAACTGG GCATCACTAA   1020
   GGTCTTCAGC AATGGGGCTG ACCTCTCCGG GGTCACAGAG GAGGCACCCC TGAAGCTCTC   1080
   CAAGGCCGTG CATAAGGCTG TGCTGACCAT CGACGAGAAA GGGACTGAAG CTGCTGGGGC   1140
   CATGTTTTTA GAGGCCATAC CCATGTCTAT CCCCCCCGAG GTCAAGTTCA ACAAACCCTT   1200
   TGTCTTCTTA ATGATTGAAC AAAATACCAA GTCTCCCCTC TTCATGGGAA AAGTGGTGAA   1260
   TCCCACCCAA AAATAACTGC CTCTCGCTCC TCAACCCCTC CCCTCCATCC CTGGCCCCCT   1320
   CCCTGGATGA CATTAAAGAA GGGTTGAGCT GG                                 1352

## Description

This invention relates to the development of fungal strains (eg of *Saccharomyces cerevisiae*) which express heterologous proteins at levels substantially higher than the parental strain.

It is known to prepare yeast strains having improved properties, for example growth characteristics or levels of expression of heterologous polypeptides. Previous methods employed enzymatic assays or antibodies applied to electrophoretic gels. For example, EP-A-201 208 discloses a screening method for detecting super-secreting mutants of *S. cerevisiae* using a milk clotting assay in which prochymosin is expressed and secreted by the putative super-secreting mutant. The present invention provides an improved method of identifying useful mutants which have a high level of expression (and, preferably, secretion) of a heterologous polypeptide.

One aspect of the present invention provides a method of determining the relative levels of production of a heterologous polypeptide in colonies of fungal cells which are capable of expressing the heterologous polypeptide, the method comprising applying the fungal cells onto a solid medium containing a means which will selectively bind to the heterologous polypeptide, allowing the cells to express the polypeptide on the solid medium, allowing interaction in the said medium of the heterologous polypeptide and the said binding means, and visualising the resulting complexes.

By a "solid" medium, we mean one which is sufficiently solid to immobilise the yeast strains and thereby allow their products to be visualised and attributed to a given strain colony. If a liquid medium were to be used, no such attribution would be possible. A gel such as agar will usually be convenient, supplemented with appropriate nutrients.

The cells may be applied to the medium by any convenient method, such as spraying or applying with a wire, commonly called "plating out".

The selection of new fungal strains involves the production of the heterologous protein, for example human albumin (HA). The method has been found to enable large numbers of individual fungal colonies to be screened for their ability to produce elevated levels of heterologous proteins.

If the polypeptide is secreted by the yeast, then one may detect the level of production without lysing the cells. A high level of production may indicate a high level of expression intracellularly coupled with a normal ability to secrete the polypeptide, or a normal level of expression coupled with an enhanced ability to secrete the polypeptide, or raised abilities in both regards. If the polypeptide is not secreted, then the cells must be lysed (either by the action of an outside agent or as a natural consequence of the growth of the yeast). A high level of production in this situation indicates a high level of expression. We have found, although we cannot predict that it will always be true, that a good expressor of the polypeptide will, if appropriate secretion leader signals are provided, often also be a good secretor and vice versa. Certainly, the raised ability of the yeast to express and/or secrete the polypeptide does not appear to be dependent upon the polypeptide itself.

The term "human albumin" (HA) is used herein to denote material which is indistinguishable from human serum albumin (HSA) or which is a variation or fragment thereof, for example forms having only one or a few amino acid residues missing or the subject of conservative substitutions, or the HSA analogues disclosed in EP-A-322 094. Generally, variants or fragments of HA will have at least 50% (preferably at least 80, 90 or 95%) of HA's ligand binding activity (for example bilirubin-binding) and at least 50% (preferably at least 80, 90 or 95%) of HA's oncotic activity, weight for weight.

The genetic material of a fungal strain with the capacity to express and secrete HA can be modified by any one of a range of standard procedures. These include exposure to electromagnetic radiation, preferably in the ultra violet (UV) range, or treatment with chemical mutagens, for example 1,2,7,8-diepoxyoctane, ethyl methanesulphonate (EMS), N-methyl-N-nitro-N-nitrosoguanidine (NTG) and 4-nitro-quinoline N-oxide (NQO), which modify the DNA sequences contained within the host. Some of these mutations are either lethal mutations or abolish the expression and/or secretion of the HA. Some mutations have the capacity to elevate the expression and secretion of HA. Clones harbouring these advantageous mutations can be detected and isolated if the cells are spread onto a solid nutrient medium, containing a human albumin (HA) antibody, in such a way as to separate individual cells from each other. When cultured at 30°C for between 72 and 96 hours isolated colonies can be observed on the nutrient medium. Ideally, each colony arises from a single cell. Colonies with the capacity to express and secrete HA are detected by an opaque halo which appears around the colony. This forms due to an interaction between the secreted HA and the anti-HA antibody. The size of the halo varies in proportion to the amount of HA secreted by the colony.

The HA coding region is preferably contained within a hybrid plasmid comprising a promoter sequence, a DNA sequence coding for HA which is under transcriptional control of the promoter and a DNA sequence containing eukaryotic transcription termination signals. The hybrid plasmid preferably also contains additional DNA sequences which perform other functions, for example in the propagation of the cells transformed with the plasmids. The "disintegration" vectors of EP-A-286 424 are examples of the hybrid plasmids which may be used.

A selective gene marker may be used to facilitate the selection of transformants due to the phenotypic expression of the marker. Suitable markers for fungi are particularly those expressing antibiotic resistance or, as in the case of auxotrophic yeast mutants, genes which complement host lesions. Corresponding genes confer, for example, resistance to the antibiotic cycloheximide or provide for prototrophy in an auxotrophic yeast mutant, for example the *URA1, URA3, LEU2, HIS4, HIS3, TRP5* and *TRP1* genes.

Antibodies may be produced and labelled in known ways, for example as disclosed in Chard "An Introduction to Radioimmunoassay and Related Techniques" (Elsevier 1982). The antibodies may be polyclonal or monoclonal.

Particularly if the heterologous polypeptide is not HA, it may be possible to visualise the polypeptide by means other than an antibody. For example, the polypeptide may have a high affinity for a cofactor or prosthetic group (eg biotin, haem Fe³⁺, NAD or FAD) or some natural ligand such as a cell surface receptor which can be labelled or tagged in known ways.

Suitable promoters for the secretion of HA include those associated with the phosphoglycerate kinase (*PGK1*) gene, *GAL1*and *GAL10* genes, *CYC1,* acid phosphatase (*PHO5*), *ADH1, ADH2*, MFα-1 pheromone, the promoter of GB-A-2 196 635 and EP-A-424 117. The preferred promoter is that of the *PRB1* gene.

The structural gene, *PRB1,* for the *Saccharomyces cerevisiae* vacuolar endoprotease B has been isolated by Moehle *et al* (1987) *Genetics* **115**, 255-263, on two *prb1* complementing plasmids called MK4 and FP8. When the yeast *Saccharomyces cerevisiae* is grown on glucose in shake flask culture, very little protease B activity is detected until the cells have catabolised the glucose and are utilising the ethanol accumulated during growth (Saheki, T. and Holzer, H. (1975) *Biochem. Biophys. Acta 384,* 203-214; Jones *et al.* (1986) UCLA Symp. Mol. Cell Biol. New Ser. *33*, 505-518). This is believed to be a consequence of a transcriptional control mechanism which represses mRNA accumulation until the glucose has been exhausted and the culture enters the diauxic plateau (Moehle *et al.* (1987) *Genetics 115*, 255-263. Studies with protease B *(prb1⁻)* deficient mutants implicate protease B in the protein degradation that occurs when negative cells are starved of nitrogen and carbon (Wolf, D. and Ehmann, C. (1979) *Eur. J. Biochem. 98*, 375-384; Zubenko, G. and Jones, E. (1981) *Genetics 97*, 45-64).

The DNA sequence of the *PRB1* gene has been reported, as has 150bp of the *PRB1* promoter (Moehle *et al.* (1987) *Mol. Cell. Biol. 7,* 4390-4399). A more extensive DNA sequence of the *PRB1* promoter is also available as an entry in the Genbank database, accession number M18097, locus YSCPRB1.

The whole of the *PRB1* promoter may be used, or a smaller portion thereof, as may readily be determined. For example, the roughly lkbp sequence extending upstream from the start codon to the *Sna*B1 site is effective.

A suitable promoter may be located on a cloning vector or an expression vector adjacent a restriction site such that a heterologous coding sequence may be located downstream of the promoter and in correct reading frame in relation to a translational start codon. The start codon may be provided on the vector (eg immediately 3' to the promoter) or it may be inserted as a 5' end of the heterologous coding sequence. A linker may be provided between the promoter of the invention and the start codon, if desired. Preferably, the DNA construct is provided at both ends with synthetic oligonucleotide linkers which allow insertion and cloning of the construct in a cloning vector. The promoter, the DNA coding sequence and the fungal transcription termination signals are operably linked to each other, ie they are juxtaposed in such a manner that their normal functions are maintained. Thus, the array is such that the expression control sequence effects proper expression of the coding sequence and the transcription termination signals effect proper termination of transcription and polyadenylation. The junction of these sequences is preferably effected by means of synthetic oligonucleotide linkers which may carry the recognition sequence of an endonuclease.

Hybrid vectors may be used having one or multiple DNA inserts each comprising a suitable promoter, a DNA segment consisting of a DNA sequence coding for a desired polypeptide which DNA segment is under transcriptional control of said promoter, and a DNA sequence containing eukaryotic transcription termination signals.

Hybrid plasmids may also be used which, apart from the expression control sequence, the above DNA segment and the sequence containing transcription termination signals, contain additional DNA sequences which are inessential or less important for the function of the promoter, ie for the expression of the desired polypeptide, but which perform important functions, for example in the propagation of the cells transformed with said hybrid plasmids. The additional DNA sequences may be derived from prokaryotic and/or eukaryotic cells and may include chromosomal and/or extra-chromosomal DNA sequences. For example, the additional DNA sequences may stem from (or consist of) plasmid DNA, such as bacterial or eukaryotic plasmid DNA, viral DNA and/or chromosomal DNA, such as bacterial, yeast or higher eukaryotic chromosomal DNA. Preferred hybrid plasmids contain additional DNA sequences derived from bacterial plasmids, especially *Escherichia coli* plasmid pBR322 or related plasmids, bacteriophage, yeast 2 µ plasmid, and/or yeast chromosomal DNA.

In the preferred hybrid plasmids, the additional DNA sequences carry a yeast replication origin and a selective genetic marker for yeast. Hybrid plasmids containing a yeast replication origin, eg an autonomously replicating segment (ars), are extrachromosomally maintained within the yeast cells after transformation and are autonomously replicated upon mitosis. Hybrid plasmids containing sequences homologous to yeast 2 µ plasmid DNA can be used as well. These hybrid plasmids may be integrated by recombination into 2 µ plasmids already present within the cell or may replicate autonomously. The integration vectors of EP-A-251 744 or the "disintegration" vectors of EP-A-286 424 may be used.

Advantageously, the additional DNA sequences which are present in the hybrid plasmids also include a replication origin and a selective genetic marker for a bacterial host, especially *Escherichia coli.* There are useful features which are associated with the presence of an *E. coli* replication origin and an *E. coli* marker in a yeast hybrid plasmid. Firstly, large amounts of hybrid plasmid DNA can be obtained by growth and amplification in *E. coli* and, secondly, the construction of hybrid plasmids is conveniently done in *E. coli* making use of the whole repertoire of cloning technology based on *E. coli. E. coli* plasmids, such as pBR322 and the like, contain both *E. coli* replication origin and *E. coli* genetic markers conferring resistance to antibiotics, for example tetracycline and ampicillin, and are advantageously employed as part of the yeast hybrid vectors.

The hybrid vectors may contain one or multiple DNA inserts each comprising *inter alia* the expression control sequence and the DNA sequence encoding the desired protein. If the hybrid vectors contain multiple DNA inserts, for example 2 to 4 DNA inserts, these can be present in a tandem array or at different locations of the hybrid vector. Preferred hybrid vectors contain one DNA insert or DNA inserts in a tandem array. The DNA inserts are especially head to tail arranged.

The hybrid plasmids are prepared by methods known in the art. The process for the preparation of the hybrid vectors comprises introducing one or multiple DNA constructs containing a promoter of the invention, a DNA segment consisting of a DNA sequence coding for a desired polypeptide which DNA segment is under transcriptional control of said expression control sequence, and a DNA sequence containing fungal transcription termination signals, as such or introducing the components of said DNA constructs successively in the predetermined order into a vector DNA.

The construction of the hybrid plasmids is performed applying conventional ligation techniques. The components of the plasmids are linked through common restriction sites and/or by means of synthetic linker molecules and/or by blunt end ligation.

Fungal cells useful in the methods of the invention include the genera *Pichia, Saccharomyces, Kluyveromyces, Candida, Torulopsis, Hansenula, Schizosaccharomyces, Citeromyces, Pachysolen, Debaromyces, Metschunikowia, Rhodosporidium, Leucosporidium, Botryoascus, Sporidiobolus, Endomycopsis,* and the like. Preferred genera are those selected from the group consisting of *Pichia, Saccharomyces, Kluyveromyces, Yarrowia* and *Hansenula,* because the ability to manipulate the DNA of these yeasts has, at present, been more highly developed than for the other genera mentioned above. Examples of *Saccharomyces* are *Saccharomyces cerevisiae, Saccharomyces italicus* and *Saccharomyces rouxii. Examples of Kluyveromyces* are *Kluyveromyces fragilis* and *Kluyveromyces lactis.* Examples of *Hansenula* are *Hansenula polymorpha, Hansenula anomala* and *Hansenula capsulata. Yarrowia lipolytica* is an example of a suitable *Yarrowia species. Aspergillus niger* is an example of a filamentous fungus.

It has been demonstrated that fungal cells of the genera *Pichia, Saccharomyces, Kluyveromyces, Yarrowia* and *Hansenula* can be transformed by enzymatic digestion of the cells walls to give spheroplasts; the spheroplasts are then mixed with the transforming DNA and incubated in the presence of calcium ions and polyethylene glycol, then transformed spheroplasts are regenerated in regeneration medium.

Methods for the transformation of *S. cerevisiae* are taught generally in EP 251 744, EP 258 067 and WO 90/01063.

The transformation of yeast with the hybrid vectors may be accomplished according to the method described by Hinnen *et al* [*Proc. Natl. Acad. Sci. USA* **75**, 1929 (1978)]. This method can be divided into three steps:
(1) Removal of the yeast cell wall or parts thereof using various preparations of glucosidases, such as snail gut juices (e.g. Glusulase^{R} or Helicase^{R}) or enzyme mixtures obtained from microorganisms (eg Zymolyase^{R}) in osmotically stabilized solutions (eg 1 M sorbitol).
(2) Treatment of the "naked" yeast cells (spheroplasts) with the DNA vector in the presence of PEG (polyethylene-glycol) and Ca⁺ ions.
(3) Regeneration of the cell wall and selection of the transformed cells in a solid layer of agar. This regeneration is conveniently done by embedding the spheroplasts into agar. For example, molten agar (about 50°C) is mixed with the spheroplasts. Upon cooling the solution to yeast growth temperatures (about 30°C), a solid layer is obtained. This agar layer is to prevent rapid diffusion and loss of essential macromolecules from the spheroplasts and thereby facilitates regeneration of the cell wall. However, cell wall regeneration may also be obtained (although at lower efficiency) by plating the spheroplasts onto the surface of preformed agar layers.

Preferably, the regeneration agar is prepared in a way to allow regeneration and selection of transformed cells at the same time. Since yeast genes coding for enzymes of amino acid biosynthetic pathways are generally used as selective markers (-*supra*), the regeneration is preferably performed in yeast minimal medium agar. If very high efficiencies of regeneration are required the following two step procedure is advantageous:
(1) regeneration of the cell wall in a rich complex medium, and
(2) selection of the transformed cells by replica plating the cell layer onto selective agar plates.

When the DNA vector is a linear DNA vector used for transforming eukaryotic host cells, transformation is preferably done in the presence of a second vector containing a selective marker for yeast. This cotransformation allows enrichment for those host cells which have taken up DNA that cannot be directly selected for. Since competent cells take up any type of DNA a high percentage of cells transformed with a selective vector will also harbour any additional DNA (such as the above linear DNA vector). The transformed host cells can be improved in production of the desired polypeptide by mutation and selection using methods known in the art. The mutation can be effected, for example, by U.V. irradiation or suitable chemical reagents. Strains which are deficient in protease A and B are particularly preferred; such strains are generally available.

The transcription termination signal can be the 3′ flanking sequence of a eukaryotic gene which contains proper signals for transcription termination and polyadenylation. Suitable 3′ flanking sequences may, for example, be those of the gene naturally linked to the expression control sequence. Alternatively, they may be different. Preferably, the termination signal is that of the *Saccharomyces cerevisiae ADH1* gene.

A second aspect of the invention provides a process for obtaining a fungal strain capable of expressing and secreting a heterologous polypeptide, the process comprising (1) exposing fungal cells to a mutagen to create mutants, (2) determining the relative levels of secretion of a heterologous peptide by a method according to any one of Claims 1 to 6, and (3) isolating a strain which expresses a desired level of the heterologous polypeptide, the level of expression being higher than the said fungal cells.

A third aspect of the invention provides a process of fermenting the improved fungal strain and obtaining a peptide expressed thereby. The peptide (which term includes oligopeptides and polypeptides) may be the same as or different from the polypeptide expressed in the strain selection stage. If different, then clearly a suitable expression means (suitable promoter and coding region etc) must be present as is well known. Usually, the expression means for the original polypeptide will be removed, before, during or after the inclusion of the new expression means.

The heterologous peptide may be fibronectin or a portion thereof (for example the collagen or fibrin-binding portions described in EP 207 751), urokinase, pro-urokinase, the 1-368 portion of CD4 (D Smith *et al* (1987) *Science* **328**, 1704-1707) platelet derived growth factor (Collins *et al* (1985) *Nature* **316**, 748-750), transforming growth factor β (Derynck *et al* (1985) *Nature* **316**, 701-705), the 1-272 portion of Von Willebrand's Factor (Bontham *et al, Nucl. Acids Res.* **145** 7125-7127), the Cathepsin D fragment of fibronectin (585-1578), α₁-antitrypsin, plasminogen activator inhibitors, factor VIII, α-globin, β-globin, myoglobin, nerve growth factor LACI (lipoprotein-associated coagulation inhibitor), lactoferrin or platelet-derived endothelial cell growth factor (PDECGF) or a conservative variant of any of these. The polypeptide may also be a fusion of HSA or an N-terminal portion thereof and any other polypeptide, such as those listed above.

The peptide is preferably initially expressed as a fusion with a secretory leader sequence. This may be, for example, the natural HA leader, the leader from the *Saccharomyces cerevisiae* MFα-1, the *Kluyveromyces lactis* killer toxin leader, a fusion between the natural HA leader and the MFα-1 leader sequence, or a fusion between the *Kluyveromyces lactis* killer leader and the MFα-1 leader sequence. Thus, at least in yeast, the leader may be either of the following leader sequences:- or or conservatively modified variations of either sequence, as described in WO 90/01063.

The fermentation of the transformed fungus and the separation of the desired peptide product are carried out by known means, for example those described in "Yeast Biotechnology" Eds. D R Berry, I Russell & G G Stewart, Allen & Unwin.

The mutant detection methods of the invention and novel promoters can be used with *S. cerevisiae,* other yeast species such as *Schizosaccharomyces pombe* or *Kluyveromyces lactis,* or other fungi such as *Aspergillus* spp.

Preferred aspects of the invention will now be described by way of example and with reference to the accompanying drawings, in which:
Figures 1 to 7 are respective plasmid maps of pAYE333, pAYE334, pAYE328, pAYE335, pAYE309, pSAC35 and pAYE316;
Figure 8 is a northern blot which describes the accumulation of HA mRNA observed in shake flask culture for two strains, DB1 pAYE316 and DS65 pAYE316, RNA being extracted from cells in A) mid-logarithmic and B) late logarithmic growth phases;
Figure 9 shows the construction of plasmids pDBP1 and pDBP2;
Figures 10 to 14 are respective plasmid maps of pDBP5, pDBP6, pDBP7, pDBA1 and pDBA2.

Standard recombinant DNA procedures are as described by Maniatis *et al*. (1982) "Molecular Cloning: A laboratory manual" (Cold Spring Harbor laboratory, Cold Spring Harbor, New York) and in the second edition thereof (Sambrook *et al*, 1989).

### Example 1: Preparation of HA secretion plasmid

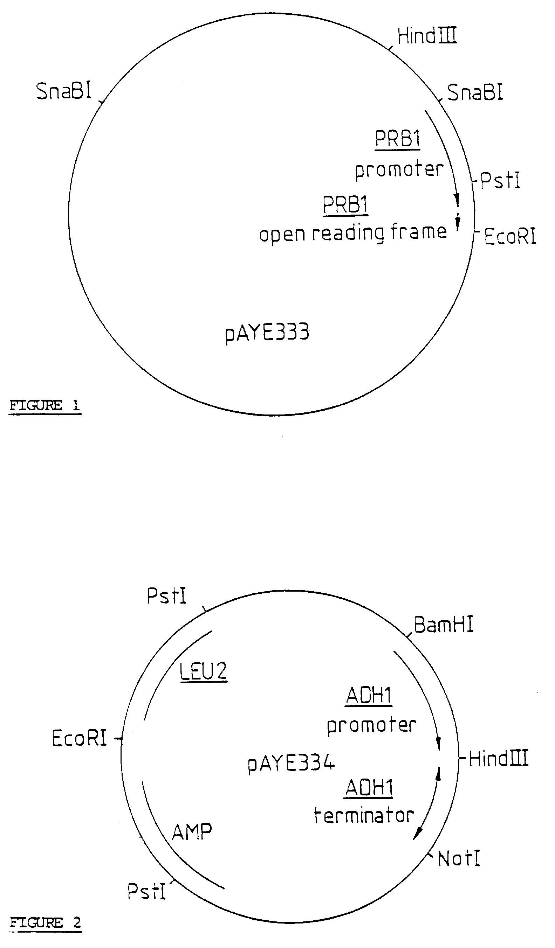

The 1.440kbp *Hin*dIII-*Eco*RI DNA fragment (**SEQ1**) containing the protease B promoter was cloned into the polylinker of the M13 bacteriophage mp18. (Yanish-Perron *et al*. (1985) *Gene 33*, 103-119), generating plasmid pAYE333 (Figure 1). Plasmid pAYE333 was linearised by partial digestion with *SnaB*l and the double stranded oligonucleotide linker 1 inserted by ligation at the *SnaBl* site within the PRB1 promoter.

This generates a *Not*I restriction site at the 5' end of the protease B promoter. The promoter element was further modified by site directed mutagenesis (oligonucleotide direct *in vitro* mutagenesis system-Version 2, Amersham) according to the manufacturer's instructions. Mutagenesis with the 31-mer oligonucleotide introduces a *Hin*dIII restriction site close to the ATG translation initiation codon:

Plasmid pAAH5 (Goodey *et al*. 1987: In *Yeast Biotechnology,* 401-429, Edited by Berry, D.R., Russell, I. and Stewart, G.G. Published by Allen and Unwin) was linearised by partially digesting with *Bam*HI. The 5' protruding ends were blunt-ended with T4 DNA polymerase and dNTPs and ligated with the double-stranded oligonucleotide Linker 1. A recombinant plasmid pAYE334 (Figure 2) was selected in which a *Not*I restriction site had replaced the *Bam*HI site at the 3' end of the *ADH*I terminator.

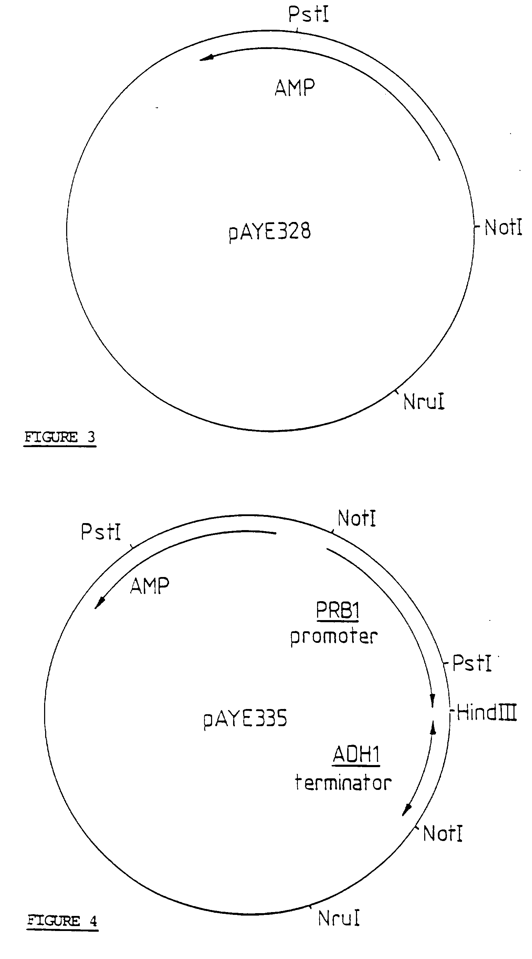

Plasmid pAT153 (Twigg & Sherratt (1980) *Nature* **283**, 216-218) was digested with *Eco*RI/*Bam*HI and the larger 3.36 kbp DNA fragment purified. The 5' protruding ends were blunt-ended with T4 DNA polymerase and dNTPs and recircularised with the double-stranded oligonucleotide Linker 1, generating plasmid pAYE328 (Fig 3).

The 0.8kbp *Not*I-*Hin*dIII modified protease B promoter sequence was placed upstream of the 0.45kbp *Hin*dIII-*Not*I *ADH*I transcription terminator on the pAT153 based plasmid pAYE328 to generate pAYE335 (Figure 4).

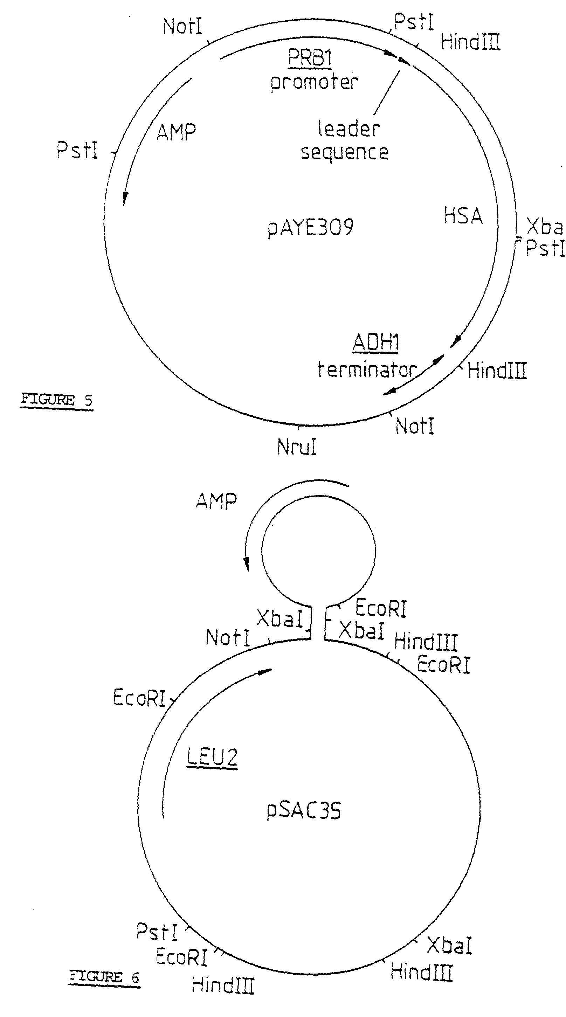

Plasmid pAYE309 (Figure 5) was constructed by a three way ligation between *Hin*dIII linearised pAYE335 (Figure 4), the double stranded oligonucleotide Linker 2 (the 5′-3′ strand of which constitutes **SEQ5**) and a 1.9 kbp HA cDNA fragment liberated from *Xho*I linearised mp 19.7 (EP-A-201 239), blunt ended with S1 nuclease and then digested with *Hin*dIII. The ATG which is marked indicates the start of an open reading frame for the fusion leader secretion sequence.

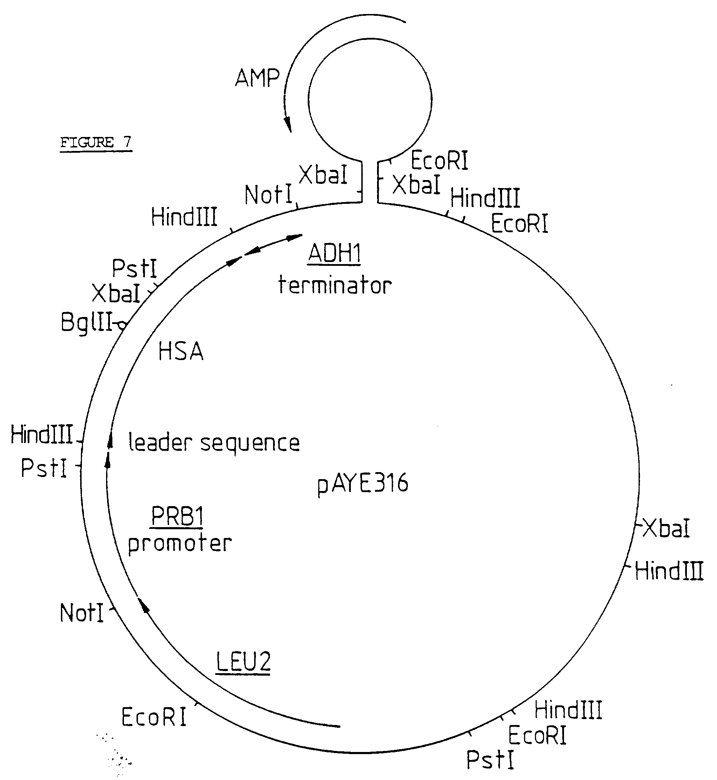

The 3.2 kbp *Not*I restriction fragment created in plasmid pAYE309 was then transferred to a suitable yeast replicating vector which contains a unique *Not*I restriction site (for example pSAC35, Figure 6) to create a plasmid pAYE316 (Figure 7).

Plasmid pSAC35 is a derivative of pSAC3 described by Chinery and Hinchliffe (1989) *Curr. Genet. 16,* 21-25 and EP286424. The *LEU*2 selectable marker is a 1.95 kbp *Sal*I-*Hpa*I fragment from YEp13, (Broach, J.R., *et al* (1979) Cell *16*, 827-839) inserted into the *Sna*BI site of pSAC3. The *LEU*2 gene possesses a unique *Tth*111 I site. Following digestion with this enzyme the 5′ protruding ends were filled in by treatment with T4 DNA polymerase I. Insertion of the *Not*I recognition site to generate pSAC35 was achieved by ligating the blunt end linearised DNA with the double stranded oligonucleotide Linker 1.

Plasmid pAYE316 was introduced into the *Saccharomyces cerevisiae* strain DB1 (*MATa, leu2,* [cir°]) by the method described by Hinnen *et al*, (1978) P.N.A.S. **75**, 1929. Transformants were selected on a minimal medium lacking leucine (Yeast nitrogen base, Difco). When transformants were grown for 72 hours at 30°C, 200 rpm in 50 ml flasks containing either 10 ml of complex (YEP, 1% (w/v) yeast extract, 2% (w/v) bactopeptone and 2% (w/v) sucrose), or defined (0.15% (w/v) yeast nitrogen base without amino acids and ammonium sulphate, 0.5% (w/v) ammonium sulphate, 0.1 M citric acid/Na₂HPO₄.12H₂O pH6.5, 2% (w/v) sucrose) liquid medium, HA could be detected in the cell free culture supernatant by SDS-polyacrylamide gel electrophoresis and/or by rocket immunogelelectrophoresis.

### Example 2: Production of super-secreting strains using HSA

Mutant strains of DB1 cir° (pAYE316) were produced by methods known in the art. Briefly DB1 cir° (pAYE316) was grown in defined media to an optical density OD₆₀₀ 0.5-1.0, (mid-logarithmic growth phase). 20 ml of the culture was centrifuged, the supernatant discarded and the cells resuspended in 20 ml defined medium lacking sucrose. The cells were then treated with one of a number of chemical mutagens available so that a survival rate of between 10-30% was achieved. Mutated cells were then spread onto YEP, 2% (w/v) sucrose, 1% (w/v) agarose plates containing 2.5% (v/v) rabbit anti-HA antibody (Cambio. Cambridge, UK) and incubated at 30°C for at least 72 hours. The number of individual cells applied to each plate was adjusted so that between 1 and 2 colonies developed per cm. As the colonies grew in size an opaque precipitation halo developed around the colonies. Mutant strains of DB1 cir° (pAYE316) secreting elevated levels of HA into the medium were detected directly by an increase in the size of the precipitin halo. Those colonies producing the largest halos were identified, subcultured onto defined medium agar plates and assessed for their ability to secrete HA in complex and defined liquid media as described above.

When an overproducing strain was identified, the location of the mutation (chromosomal versus episomal) was established by curing the strain of the plasmid and retransforming the [cir°] mutant strain back to leucine prototrophy with the plasmid pAYE316. If the mutation is localised in the genome then the retransformed strain will retain the capacity to secrete HA at elevated levels.

The precipitin halo which develops is generally pale and hazy. Preferably, therefore, the medium used is clear and thus agarose is preferable to agar. The concentration of the solid medium (if not clear) and the density of cells deposited thereon can be so chosen as to yield the most clear result.

If the heterologous polypeptide is not secreted, it may nevertheless still be detected either by deliberately lysing the cells mechanically, chemically or enzymatically or by allowing autolysis to occur.

In an alternative embodiment, which is particularly useful when only low levels of expression are obtained, the cells are plated onto agarose or another suitable medium and then dried down onto a hydrophilic plastics sheet such as "Gelbond" (Trademark, Pharmacia, Sweden). The antibody-HA complexes can then be visualised with, for example, a Coomassie Blue (Trademark) stain to reveal blue halos.

An oversecreting strain can be mutated again. Successive rounds of mutation substantially increase the productivity of the strain. In this way a series of mutated strains have been developed (Table 1). All the mutations were chromosomal in origin. DS37 cir° (pAYE316) was originally observed to be an over-secreting mutant strain of DBI cir° (pAYE316). However, results subsequently showed that this strain did not secrete HA at significantly higher levels than DB1 cir° (pAYE316) when assessed in complex liquid medium as described above.

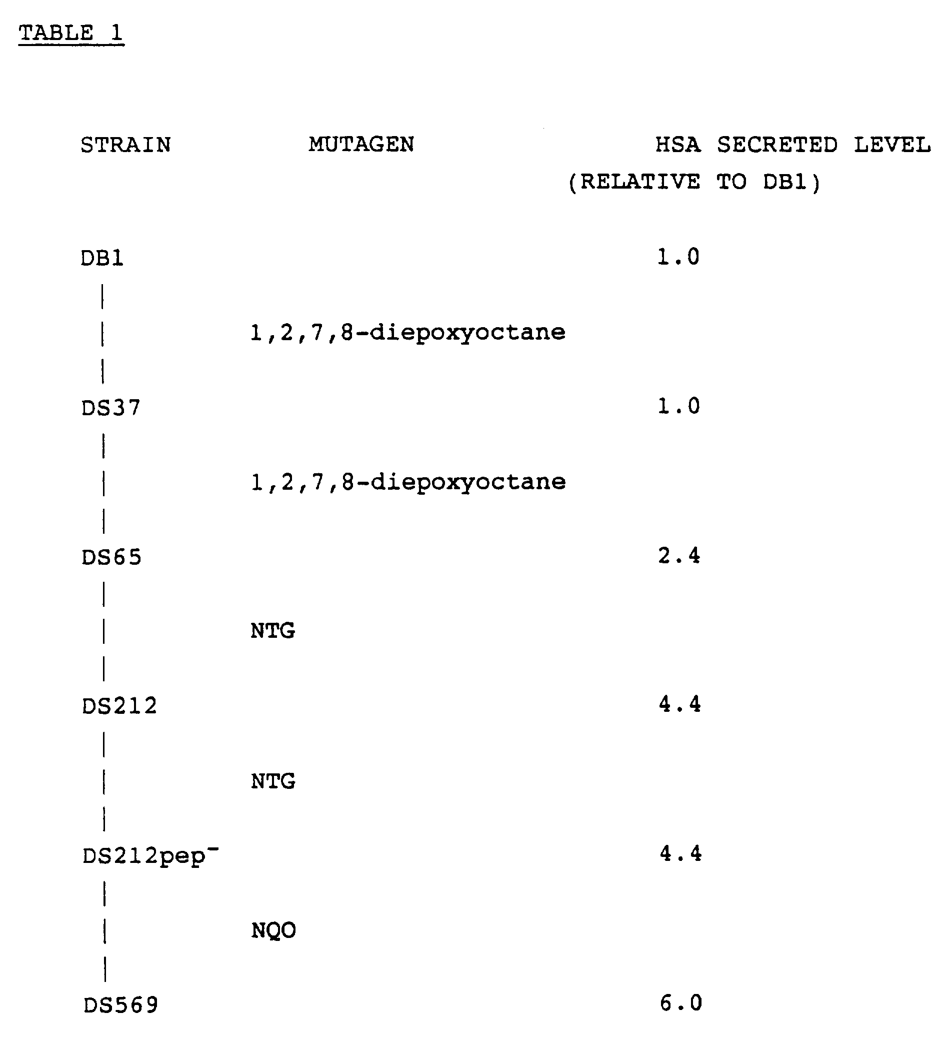

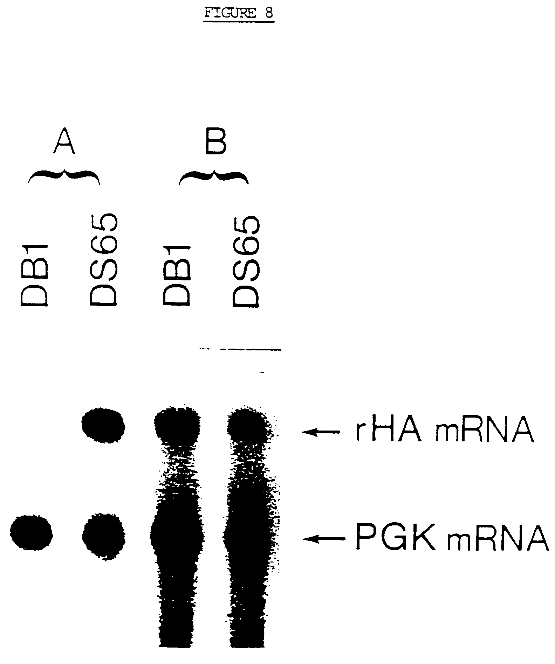

The precise genetic loci of the lesions are not known. However the phenotypic effects of two of the mutations have been partially characterised. The steady state levels of HA mRNA in the parental strain DB1 cir° (pAYE316) mirror the levels of protease B mRNA observed during growth in shake flask culture (Moehle (1987) *Genetics 115,* 255-263). The mutation which results in the increased productivity of strain DS65 cir° (pAYE316) apparently allows constitutive expression of HA mRNA from the protease B promoter during the logarithmic growth phase (Figure 8).

A second mutation apparently abolishes synthesis of active protease A. The strain in which this mutation was first identified, DS212pep⁻ cir° (pAYE316), secretes HA at levels indistinguishable from its parent strain DS212 cir° (pAYE316).

### Example 3: Intracellular expression of PAI-2

A lambda gt11 cDNA library constructed from mRNA isolated from 4-phorbol-12-myristate-13-acetate stimulated cells of the human monocyte-like histiocytic lymphoma cell line U937 (obtained from Clontech Laboratories Inc.) was used as a source of plasminogen activator inhibitor type 2 (PAI-2) cDNA. The library was screened using radioactively labelled oligonucleotide probes corresponding to the DNA sequences encoding the N-terminus (oligo 1) and C-terminal end (amino acids 400-410) of the PAI-2 protein, respectively.

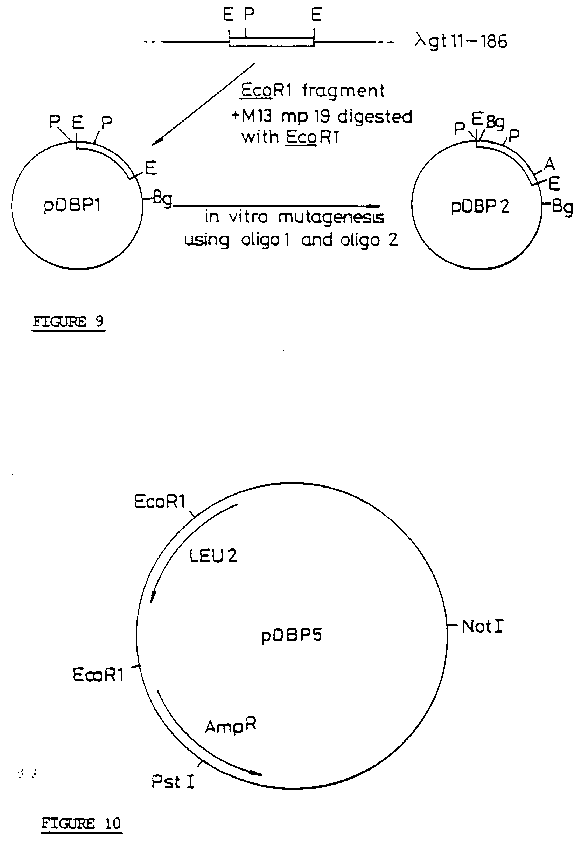

From the putative positive clones was selected one clone (lambda gt11-186) which appeared to contain the entire PAI-2 coding region. This was confirmed by sequence analysis of the DNA insert in this clone (**SEQ8**) following transfer to M13mp19 to form pDBP1 (Figure 9). In **SEQ8**, the ATG at position 42 marks the start of the coding region and the TAA at position 1287 is a stop codon.

To facilitate insertion into expression vectors, restriction enzyme recognition sites were created at the 5′ and 3′ ends of the PAI-2 gene. A *Bgl*II site was created at the 5′ end of the gene using the oligonucleotide primer to create a mutation in the third position of the second codon as shown below:- changed to:- (**SEQ11** is the start of the protein sequence shown). An *Afl*II site was created at the 3′ end of the gene using the oligonucleotide primer: to create mutations in the third position of the last codon (proline) and in the first base after the stop codon as shown below:- changed to: (**SEQ15** is the peptide encoded by the C-terminal region shown). These two oligonucleotides were annealed to single stranded pDBP1 and then used in an *in vitro* mutagenesis procedure (Amersham plc) carried out according to the manufacturer's recommendations. A clone derived from this procedure and with the correct changes was designated pDBP2 (Figure 9).

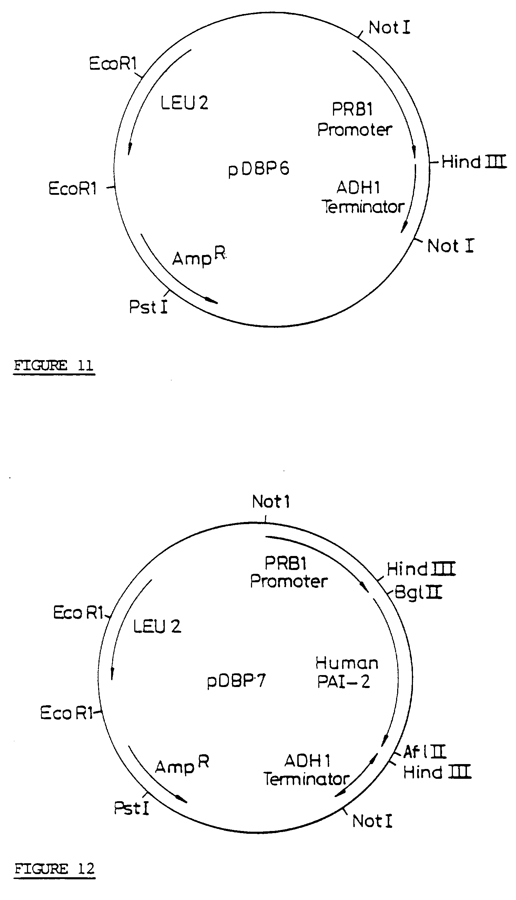

The large 6.38 kbp *Hin*dIII-*Bam*HI fragment from the yeast *E.coli* shuttle vector pJDB207 (Beggs, J.D. 1981 *Molecular Genetics in Yeast*, Alfred Benzon Symposium *16*, 383-395) was treated with the Klenow fragment of *E. coli* DNA polymerase to create flush ends and ligated with the double stranded oligonucleotide Linker 1 (Example 1) to generate plasmid pDBP5 (Figure 10). The 1.25 kbp *Not*I Protease B promoter/*ADH*1 terminator cassette from plasmid pAYE335 (Figure 4) was introduced into the unique *Not*I site of plasmid pDBP5 generating pDBP6 (Figure 11).

Two double stranded oligonucleotide linkers were used to allow the insertion of a human PAI-2 cDNA into the expression plasmid pDBP6. Linkers 3 and 4 were ligated with the 1.34kbp *Bgl*II-*Afl*II PAI-2 cDNA from pDBP2 (Figure 9) into *Hin*dIII linearised pDBP6 generating plasmid pDBP7 (Figure 10).

Stable maintenance of pDBP7 by the *S. cerevisiae* strains DB1 cir° and DS569 cir° can not be accomplished until *trans* acting functions present on the native 2µ plasmid (Futcher, A.B., (1988) *Yeast 4*, 27-40) have been introduced. This was achieved by co-transforming DB1 cir° and DS569 cir° with pSAC3 (Chinery, S.A. and Hinchliffe, E. (1989) *Current Genetics 16,* 21-25, EP286424) and pJDB207 and selecting for transformants on minimal media lacking leucine. Curing of DB1 cir° (pSAC3/pJDB207) and DS569 cir° (pSAC3/pJDB207) of the pJDB207 plasmid results effectively in cir⁺ derivatives of the original strains.

DB1 cir° (pSAC3) and DS569 cir° (pSAC3) were re-transformed to leucine prototrophy with plasmid pDBP7 and transformants selected on minimal media lacking leucine. DS569 cir° (pSAC3), DS569 cir° (pSAC3/pDBP7), DB1 cir° (pSAC3) and DB1 cir° (pSAC3/pDBP7) were grown for 72 hours in 10ml complex media (1% (w/v) yeast extract; 2% (w/v) bactopeptone and 2% (w/v) sucrose) in shake flask culture at 200rpm, 30°C. Cells were harvested by centrifugation and lysed and the soluble protein extracts analysed by SDS-polyacrylamide gel electrophoresis. An extra protein band of 46kD is only observed in the soluble protein extracts of those cultures containing the PAI-2 expressing plasmid pDBP7. This extra protein band is of the predicted molecular weight for full length human PAI-2 and was shown by Western blot analysis to react with anti-PAI-2 antibodies, thereby verifying its identity as PAI-2. The strain DS569 cir° (pSAC3/pDBP7), which was originally selected on the basis that it secreted elevated levels of human albumin into the culture supernatant, accumulates human PAI-2 at levels at least ten-fold higher than its ancestral strain DB1 cir° (pSAC3/pDBP7).

### Example 4: Intracellular expression of α₁AT

A human α₁-antitrypsin cDNA, identical in sequence to that described in the Genbank database, accession number K01396, locus HUMA1ATM, was amplified by Polymerase Chain Reaction (PCR) according to the manufacturer's instructions.

The sequence is reproduced here as **SEQ19**.

The DNA sequences of the two primary PCR oligonucleotides were: Upon amplification both the 5' and 3' termini of the α₁-antitrypsin sequence became modified as follows:

The respective encoded amino acid regions appear as **SEQ23** and **SEQ25**. (The C-terminal region indicated above appears as **SEQ27**). These modifications remove the 23 amino acid signal sequence and introduce a *Hin*dIII restriction site at the 3' end of the cDNA.

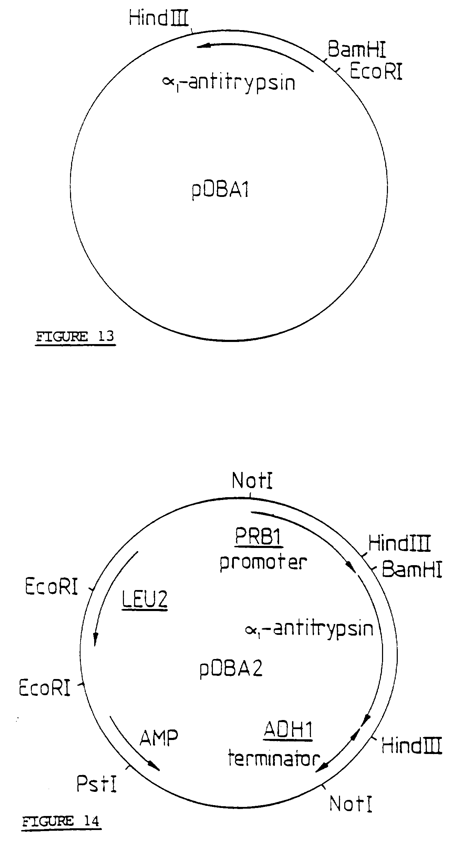

The 1.18kbp modified cDNA was purified, digested with *Hind*III and *Bam*HI inserted and cloned into the *Hin*dIII-*Bam*HI sites of M13mp19 (Yanish-Perron *et al* (1985) *Gene* 33, 103-119) generating pDBA1 (Figure 13). The integrity of human α₁-antitrypsin was confirmed by dideoxynucleotide sequencing.

A double stranded oligonucleotide linker was used to allow the insertion of the human α₁-antitrypsin cDNA into the yeast expression plasmid pDBP6 (Figure 11). Linker 5 was ligated with the 1.18kbp *Bam*HI-*Hin*dIII human α₁-antitrypsin cDNA into the *Hin*dIII linearised pDBP6 generating plasmid pDBA2 (Figure 14).

DB1 cir° (pSAC3) and DS569 cir° (pSAC3) were transformed to leucine prototrophy with plasmid pDBA2 and transformants selected on minimal medium lacking leucine. DB1 cir° (pSAC3/pDBA2) and DS569 cir° (pSAC3/pDBA2) were grown for 72 hours in 10ml complex medium (1% (w/v) yeast extract; 2% (w/v) bactopeptone and 2% (w/v) sucrose) in shake flask culture at 200rpm, 30°C. Cells were harvested by centrifugation and lysed and the soluble protein extracts analysed by SDS-polyacrylamide gel electrophoresis.

An intense protein band of α₁-antitrypsin is observed in the soluble protein extracts of DS569 cir° (pSAC3/pDBA2). This extra band is of the predicted molecular weight for α₁-antitrypsin. α₁-antitrypsin is also detected in the soluble protein extracts of DB1 cir° (pSAC3/pDBA2). DS569 cir° (pSAC3/pDBA2) accumulates α₁-antitrypsin at levels at least tenfold higher than its ancestral strain DB1 cir° (pSAC3/pDBA2).

## Claims

1. A method of determining the relative levels of production of a heterologous polypeptide in colonies of fungal cells which are capable of expressing the heterologous polypeptide, the method comprising applying the fungal cells onto a solid medium, containing a means which will selectively bind to the heterologous polypeptide, allowing the cells to express the polypeptide on the solid medium allowing interaction in the said medium of the heterologous polypeptide and the said binding means, and visualising the resulting complexes.

2. A method according to Claim 1 wherein the said means is an antibody to the polypeptide and the said visualising step consists of observing halos of precipitated antibody/polypeptide complexes.

3. A method according to Claim 1 or 2 wherein the said medium comprises agarose.

4. A method according to any one of the preceding claims wherein the fungal cells are *Saccharomyces cerevisiae.*

5. A method according to any one of the preceding claims wherein the heterologous polypeptide is human albumin, human PAI-2 or human α₁-antitrypsin.

6. A method according to any one of the preceding claims wherein the polypeptide is secreted by the fungal cells.

7. A process for obtaining a fungal strain capable of producing a first heterologous polypeptide, the process comprising (1) exposing fungal cells to a mutagen to create mutants, (2) determining the relative levels of production of a second heterologous peptide by a method according to any one of Claims 1 to 6, and (3) isolating a strain which produces a desired level of the first heterologous polypeptide, the level of production being higher than the said fungal cells, wherein the first and second polypeptides may be the same or different.

8. A process for preparing a heterologous polypeptide comprising fermenting a fungal strain obtained by a method according to Claim 7 and purifying the first polypeptide.

9. A process according to Claim 8 wherein the fungal strain is first transformed with means to express the said first polypeptide, the first polypeptide being different from the second polypeptide of Claim 7.

10. A process according to Claim 8 or 9 wherein the first heterologous polypeptide is secreted from the fungal cells.

## Patentansprüche

1. Verfahren zur Bestimmung der relativen Produktionsmengen eines heterologen Polypeptids in Kolonien von Pilzzellen mit der Fähigkeit zur Expression des heterologen Polypeptids, wobei das Verfahren ein Aufbringen der Pilzzellen auf ein ein Mittel, das selektiv an das heterologe Polypeptid bindet, enthaltendes, festes Medium, ein Zulassen eines Wechselwirkens des heterologen Polypeptids mit dem bindenden Mittel in dem Medium und ein Sichtbarmachen der gebildeten Komplexe umfaßt.

2. Verfahren nach Anspruch 1, wobei das Mittel ein Antikörper zu dem Polypeptid ist und die Stufe des Sichtbarmachens aus einem Beobachten von Haloeffekten der ausgefallenen Antikörper/Polypeptid-Komplexe besteht.

3. Verfahren nach Anspruch 1 oder 2, wobei das Medium Agarose umfaßt.

4. Verfahren nach einem der vorhergehenden Ansprüche, wobei die Pilzzellen Saccharomyces cerevisiae sind.

5. Verfahren nach einem der vorhergehenden Ansprüche, wobei das heterologe Polypeptid Humanalbumin, Human-PAI-2 oder Human-α₁-antitrypsin ist.

6. Verfahren nach einem der vorhergehenden Ansprüche, wobei das Polypeptid durch Pilzzellen abgesondert wird.

7. Verfahren zur Gewinnung eines Pilzstamms mit der Fähigkeit zur Produktion eines ersten heterologen Polypepetids, wobei das Verfahren die folgenden Stufen umfaßt: (1) Einwirkenlassen eines Mutagens auf Pilzzellen, um Mutanten zu erzeugen, (2) Bestimmen der relativen Produktionsmengen eines zweiten heterologen Peptids im Rahmen eines Verfahrens nach einem der Ansprüche 1 bis 6 und (3) Isolieren eines Stamms, der die gewünschte Menge des ersten heterologen Polypeptids produziert, wobei die Produktionsmenge die der Pilzzellen übersteigt und wobei das erste und zweite Polypeptid gleich oder verschieden sein können.

8. Verfahren zur Herstellung eines heterologen Polypeptids durch Fermentieren eines gemäß dem Verfahren nach Anspruch 7 erhaltenen Pilzstamms und Reinigen des ersten Polypeptids.

9. Verfahren nach Anspruch 8, wobei der Pilzstamm mit Mitteln zur Expression des ersten Polypeptids zuerst transformiert wird, wobei sich das erste Polypeptid vom zweiten Polypeptid nach Anspruch 7 unterscheidet.

10. Verfahren nach Anspruch 8 oder 9, wobei das erste heterologe Polypeptid aus Pilzzellen abgesondert wird.

## Revendications

1. Procédé de détermination des taux relatifs de production de polypeptide hétérologue dans des colonies de cellules de champignon qui sont capables d'exprimer le polypeptide hétérologue, le procédé comprenant l'application des cellules de champignon sur un milieu solide contenant un moyen qui va se lier sélectivement au polypeptide hétérologue, permettant aux cellules d'exprimer le polypeptide sur le milieu solide, permettant une interaction du polypeptide hétérologue et dudit moyen de liaison dans ledit milieu, et la visualisation des complexes résultants.

2. Procédé selon la revendication 1 dans lequel ledit moyen est un anticorps du polypeptide et ladite étape de visualisation consiste en l'observation de halos de complexes d'anticorps/polypeptides précipités.

3. Procédé selon la revendication 1 ou 2 dans lequel ledit milieu comprend l'agarose.

4. Procédé selon l'une quelconque des revendications Précédentes dans lequel les cellules de champignon sont des *Saccharomyces cerevisiae.*

5. Procédé selon l'une quelconque des revendications précédentes dans lequel le polypeptide hétérologue est de l'albumine humaine, du PAI-2 humaine ou de l'α₁-antitrypsine humaine.

6. Procédé selon l'une quelconque des revendications précédentes dans lequel le polypeptide est sécrété par les cellules de champignon.

7. Processus pour l'obtention d'une lignée de champignon capable de produire un premier polypeptide hétérologue, le processus comprenant (1) l'exposition des cellules de champignon à un mutagène pour créer des mutants, (2) la détermination des taux relatifs de production d'un second peptide hétérologue par un procédé selon l'une quelconque des revendications 1 à 6, et (3) l'isolement de la lignée qui produit le taux désiré du premier polypeptide hétérologue, le taux de production étant plus élevé que lesdites cellules de champignon, dans lequel le premier et le second polypeptide peuvent être identiques ou différents.

8. Processus pour la préparation d'un polypeptide hétérologue comprenant la fermentation d'une lignée de champignon obtenue par un procédé selon la revendication 7 et la purification du premier polypeptide.

9. Processus selon la revendication 8 dans lequel la lignée de champignon est transformée d'abord par des moyens pour exprimer ledit premier polypeptide, le premier polypeptide étant différent du second polypeptide de la revendication 7.

10. Processus selon la revendication 8 ou 9 dans lequel le premier polypeptide hétérologue est sécrété à partir des cellules de champignon.
